(19) Europäisches Patentamt
European Patent Office
Office européen des brevets

(11) Numéro de publication: **0 248 734 B1**

(12) # FASCICULE DE BREVET EUROPEEN

(45) Date de publication du fascicule du brevet: **06.09.89**

(21) Numéro de dépôt: **87401244.6**

(22) Date de dépôt: **04.06.87**

(51) Int. Cl.⁴: **C07D 333/54**, C07D 307/79, C07D 409/04, A61K 31/38, A61K 31/34

(54) **Dérivés de benzo[b]thiophène et benzo[b]furannecarboxamides, leurs procédés de préparation et les médicaments les contenant.**

(30) Priorité: **05.06.86 FR 8608110**

(43) Date de publication de la demande: **09.12.87 Bulletin 87/50**

(45) Mention de la délivrance du brevet: **06.09.89 Bulletin 89/36**

(84) Etats contractants désignés: **AT BE CH DE ES FR GB GR IT LI LU NL SE**

(56) Documents cités:
**EP-A- 0 147 044**

**CHEMICAL ABSTRACTS, vol. 99, no. 25, 19 décembre 1983, page 586, no. 211859u, Columbus, Ohio, US; A.I. HASHEM: "Reactivity of some butenolides towards intramolecular and intermolecular alkylation reactions", & EGYPT. J. CHEM. 1982 (Pub. 1983), 25(6), 541-44, 47(5), 1207-1208erial scanned IR imager" 000**

**CHEMICAL ABSTRACTS, vol. 100, no. 3, 16 janvier 1984, page 482, no. 22531j, Columbus, Ohio, US; O.H. HISHMAT et al.: "Some reactions of benzofuran derivatives", & POL. J. CHEM. 1982, 56(4-5-6), 691-7**

(73) Titulaire: **RHONE-POULENC SANTE, 20, avenue Raymond Aron, F-92160 Antony(FR)**

(72) Inventeur: **Dubroeucq, Marie-Christine, 13 Villa Malleville, F-95880 Enghien-Les-Bains(FR)**
Inventeur: **Renault, Christian, 61 Rue des Mallets, F-95150 Taverny(FR)**

(74) Mandataire: **Savina, Jacques et al, RHONE-POULENC INTERSERVICES Service Brevets Pharma 25, quai Paul Doumer, F-92408 Courbevoie Cédex(FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

Dérivés de benzo[b]thiophène et benzo[b]furannecarboxamides, leurs procédés de préparation et les médicaments les contenant

La présente invention concerne des dérivés de benzo[b]thiophène et benzo[b]furannecarboxamides, leurs procédés de préparation et les médicaments les contenant.

Les composés selon l'invention peuvent être représentés par la formule :

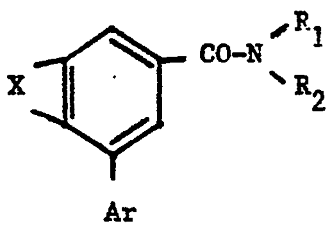

(I)

dans laquelle $R_1$ et $R_2$, identiques ou différents, représentent des groupes alkyle à chaîne droite ou ramifiée comportant 1 à 4 atomes de carbone, cycloalkylalkyle dont la partie alkyle comporte 1 à 3 atomes de carbone et la partie cycloalkyle comporte 3 à 6 atomes de carbone ou phényle,

$R_1$ et $R_2$ peuvent également former ensemble avec l'atome d'azote auquel ils sont rattachés un cycle pipéridine,

Ar représente un radical phényle, thiényle ou phényle substitué par un ou deux substituants choisis parmi les atomes d'halogène (fluor, chlore, brome), les groupes alkyle ou alcoxy comportant 1 à 4 atomes de carbone, nitro ou trifluorométhyle,

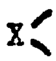 représente l'un des enchaînements suivants:

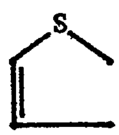 , 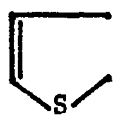 , 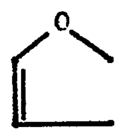 , 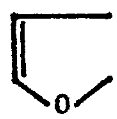

Autrement dit, les composés de formule (I) de la présente invention répondent à l'une des 4 formules ci-dessous:

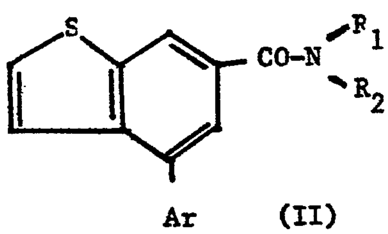

(II)

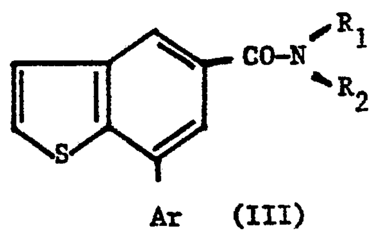

(III)

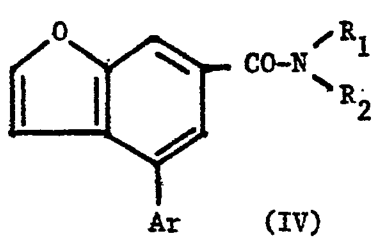

(IV)

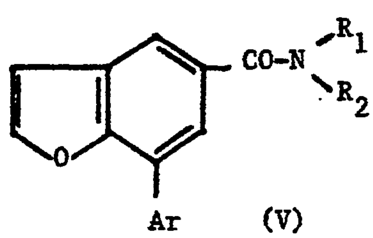

(V)

dans les formules (II), (III), (IV), et (V), Ar, $R_1$ et $R_2$ ont les mêmes significations que dans la formule (I).

Lorsque le groupe $NR_1R_2$ comporte un ou plusieurs atomes de carbone asymétriques, pour une signification donnée de Ar, $R_1$ et $R_2$, il y a plusieurs stéréoisomères correspondant à la formule plane (I). Ces divers stéréoisomères ainsi que les racémiques correspondants font partie de l'invention.

Les composés de formule (I) peuvent être préparés par action d'une amine de formule :

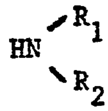 (VI)

ou un sel de cette amine, sur un composé de formule :

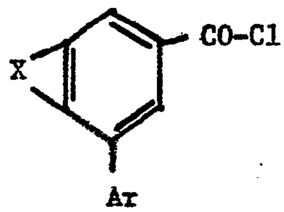 (VII)

dans lesquelles Ar, 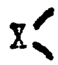 , $R_1$ et $R_2$ ont les mêmes significations que dans la formule (I).

Comme sels de l'amine de formule (VI), on peut citer le chlorhydrate ou le p-toluènesulfonate.

Cette réaction peut être réalisée selon des méthodes connues en soi, permettant de transformer un chlorure d'acide en carboxamide telles que celles décrites par C.A. BUEHLER et D.E. PEARSON, Survey of Organic Synthesis, Wiley Interscience, 1970, p. 804.

Il est particulièrement avantageux de faire réagir le chlorure d'acide de formule (VII) avec l'amine de formule (VI) en présence d'une amine tertiaire telle que la triéthylamine, au sein d'un solvant inerte tel que le toluène, le chloroforme ou le chlorure de méthylène, à une température comprise entre 20°C et la température d'ébullition du solvant. Dans le cas où l'on utilise un sel d'amine de formule (VI), il est nécessaire d'utiliser au moins deux équivalents d'amine tertiaire pour un équivalent de sel d'amine.

Les chlorures d'acide de formule (VII) peuvent être obtenus par action, sur un acide de formule :

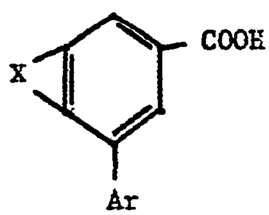 (VIII)

dans laquelle Ar et 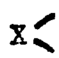
ont les mêmes significations que dans la formule (VII), d'un agent de chloruration tel que le chlorure de thionyle.

Cette réaction peut être réalisée en l'absence de solvant ou au sein d'un solvant inerte tel que le chloroforme ou le toluène, de préférence à la température d'ébullition du mélange.

Certains acides de formule (VIII) sont connus. Ce sont les acides (méthyl-4 phényl, méthoxy-4 phényl et chloro-4 phényl)-4 benzo[b]furannecarboxylique-6 (A.J. HASHEM, Journal F. Prakt. Chemie, 319(4), 689-692 (1977)).

Les autres acides de formule (VIII) peuvent être préparés par adaptation de la méthode décrite par L.S. EL ASSAL et A.H SHEHAB, J. Chem. Soc. 1658-1662, 1961 et qui consiste à chauffer à une température comprise entre 100 et 120°C un composé de formule :

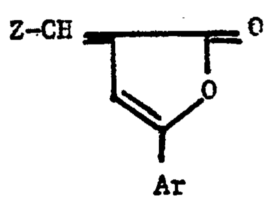 (IX)

dans laquelle Z est un radical thiényl-2, thiényl-3, furyl-2 ou furyl-3 et Ar a la même signification que dans la formule (VIII), au sein d'un mélange 50-50 en parties d'acide méthanesulfonique et d'acide acétique.

Les composés de formule (IX) peuvent être obtenus par action de thiophènecarboxaldéhyde-2 ou -3 ou de furannecarboxaldéhyde-2 ou -3 sur un dérivé de formule:

$Ar-CO-CH_2-CH_2-COOH$ (X)
dans laquelle Ar a la même signification que dans la formule (IX), en présence d'anhydride acétique et d'acétate alcalin tel que l'acétate de sodium ou de potassium, à une température de 80 à 90°C.

Les composés de formule (I) dans laquelle $R_1$ est un groupe alkyle comportant 1 à 4 atomes de carbone et $R_2$, Ar, 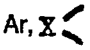

ont les mêmes significations que précédement peuvent être préparés par alkylation des composés de formule:

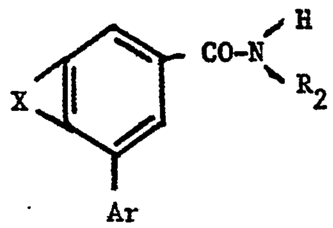

(XI)

dans laquelle $R_2$, Ar et 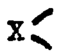

ont les mêmes significations que dans la formule (I) avec un halogènure d'alkyle de formule :
$Hal-R'_1$ (XII)
dans laquelle Hal est un atome d'halogène et $R'_1$ est un groupe alkyle comportant 1 à 4 atomes de carbone.

Cette réaction peut être effectuée selon une méthode connue en soi telle que celle décrite par A.W. JOHNSTONE et M.F. ROSE, Tetrahedron 25, 2169-73, 1979.

Un procédé avantageux consiste à opérer sous atmosphère d'azote, à la température ambiante, en présence d'une base forte telle que l'hydroxyde de potassium en poudre au sein d'un solvant tel que le diméthylsulfoxyde.

Les composés de formule (XI) peuvent être préparés par action d'une amine de formule :
$H_2NR_2$ (XII)
dans laquelle $R_2$ a les mêmes significations que dans la formule (I) sur un composé de formule (VII) dans laquelle Ar et 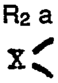

ont les mêmes significations que dans la formule (I).

Cette réaction est réalisée dans les mêmes conditions que celles mentionnées ci-dessus pour préparer les composés de formule (I) à partir des composés de formule (VII) et des amines de formule (VI).

Les stéréoisomères des composés de formule (I) dans laquelle le groupe $NR_1R_2$ comporte un ou plusieurs atomes de carbone asymétiques peuvent être obtenus par dédoublement des racémiques, par exemple, par chromatographie sur colonne chirale selon W.H. PIRKLE et Coll, asymetric synthesis, vol 1, Academic Press (1983) ou encore par synthèse à partir des précurseurs chiraux.

Les mélanges réactionnels obtenus par les divers procédés décrits précédemment sont traités suivant des méthodes classiques physiques (évaporation, extraction, distillation, cristallisation, chromatographie) ou chimiques (formation de sel et récupération de la base ou de l'acide) afin d'isoler les composés de formule (I) à l'état pur.

Les composés de formule (I) présentent des propriétés pharmacologiques intéressantes. Ces composés se lient aux récepteurs des benzodiazépines du type périphérique et sont utiles comme anxiolytiques, antiangoreux et immunomodulateurs.

L'affinité des composés de formule (I) pour les sites récepteurs des benzodiazépines du type périphérique a été déterminée en utilisant le protocole de BRAESTRUP et Coll, Proc. Natl. Acad. Sci. USA, 74, 3805 (1977) sur des membranes de rein de rat avec comme ligand le $^3$H-PK 11195 (N-méthyl N-(méthyl-1 propyl) (chloro-2 phényl)-1 isoquinoléinecarboxamide-3). Cette affinité se traduit par une constante d'inhibition (Ki) comprise entre 0,001 et 0,200 µM.

Les composés selon l'invention présentent une faible toxicité. Leur $DL_{50}$ par voie orale chez la souris est supérieure à 200 mg/kg. Les $DL_{50}$ ont été calculées après 3 jours d'observation par la méthode cumulative de J.J. REED et H. MUENCH, Amer. J. Hyg., 27, 493 (1938).

Sont particulièrement intéressants les composés de formule (I) dans laquelle $R_1$ et $R_2$, identiques ou différents, sont des groupes alkyle à chaîne droite ou ramifiée comportant 1 à 4 atomes de carbone et Ar est un radical phényle éventuellement substitué par un atome d'halogène ou un groupe nitro ou un radical thiényl-2.

Sont particulièrement interessants les composés suivants :
- N-méthyl N-(méthyl-1 propyl) phényl-4 benzo[b]furannecarboxamide-6
- N-méthyl N-(méthyl-1 propyl) (chloro-2 phényl)-7 benzo[b]thiophènecarboxamide-5
- N-méthyl N-(méthyl-1 propyl) phényl-4 benzo[b]thiophènecarboxamide-6
- N-méthyl N-(méthyl-1 propyl) (fluoro-2 phényl)-7 benzo[b]thiophènecarboxamide-5

- N,N-diéthyl (nitro-3 phényl)-7 benzo[b]thiophènecarboxamide-5
- N-méthyl N-(méthyl-1 propyl) (thiényl-2)-7 benzo[b]furannecarboxamide-5
- N-méthyl N-(méthyl-1 propyl) phényl-7 benzo[b]thiophènecarboxamide-5

Les exemples suivants, donnés à titre non limitatif, montrent comment l'invention peut être mise en pratique.

EXEMPLE 1

On chauffe à 90°C pendant 2 heures, 3g d'acide phényl-7 benzo[b]thiophènecarboxylique-5 et 2,6 $cm^3$ de chlorure de thionyle dans 30 $cm^3$ de toluène. On évapore, sous pression réduite, le solvant et le chlorure de thionyle en excés puis on ajoute au résidu 30 $cm^3$ de toluène et 5,1 $cm^3$ de triéthylamine.

On agite et ajoute, goutte à goutte, 1,23 $cm^3$ de diéthylamine. On agite encore 1 heure à température ambiante (20°C environ), évapore le toluène sous pression réduite et reprend le résidu par du chlorure de méthylène et une solution aqueuse de carbonate de potassium. On agite 5 minutes, lave la phase organique par de l'eau, la sèche sur sulfate de magnésium et l'évapore sous pression réduite. Le résidu obtenu est chromatographié sur du gel de silice en utilisant un mélange cyclohexane-éthanol-acétate d'éthyle (80-10-10 en volume) comme éluant.

Après recristallisation du résidu dans l'éther isopropylique, on obtient 2g de N,N-diéthyl phényl-7 benzo[b]thiophènecarboxamide-5 fondant à 78°C.

L'acide phényl-7 benzo[b]thiophènecarboxylique-5 peut être préparé de la manière suivante :

On chauffe à 120°C, pendant 1 heure, 44,7g de phényl-5 (thiényl-3 méthylène)-3 furanone-2, 220 $cm^3$ d'acide méthanesulfonique et 220 $cm^3$ d'acide acétique glacial. On refroidit à 40°C, coule le mélange sur de la glace pilée et extrait par du chlorure de méthylène. La phase organique est lavée à l'eau et extraite par une solution normale d'hydroxyde de sodium. La phase aqueuse basique est lavée au chlorure de méthylène, acidifiée à pH 1 par addition d'une solution aqueuse d'acide chlorhydrique et extraite au chlorure de méthylène. Le précipité qui se forme est filtré, la phase organique est séchée sur sulfate de magnésium et évaporée sous pression réduite. Le résidu d'évaporation et le précipité sont rassemblés et agités dans l'acétate d'éthyle en présence de 100g de gel de silice. La silice est éliminée par filtration et l'acétate d'éthyle évaporé sous pression réduite. On réitère cette opération pour obtenir 29 g d'acide phényl-7 benzo[b]thiophènecarboxylique-5 fondant à 234°C.

La phényl-5 (thiényl-3 méthylène)-3 furanone-2 peut être préparée de la manière suivante :

On chauffe à 80°C, pendant une nuit, 27g de thiophénecarboxaldéhyde-3, 35,8g d'acide benzoyl-3 propionique, 57 $cm^3$ d'anhydride acétique et 16,5g d'acétate de potassium fondu. On refroidit à 40°C, ajoute du chloroforme puis verse sur de la glace pilée. On extrait au chloroforme, sèche la phase organique sur du sulfate de magnésium et l'évapore sous pression réduite. Le résidu est agité 45 minutes dans de l'éther de pétole 40-60°. Après filtration et sèchage, on isole 49g de phényl-5 (thiényl-3 méthylène)-3 furanone-2 fondant à 153°C.

EXEMPLE 2

A 12 $cm^3$ de diméthylsulfoxyde sous atmosphère d'azote, on ajoute, sous agitation, 1,8g d'hydroxyde de potassium en poudre puis 2g de N-(méthyl-1 propyl) phényl-7 benzo[b]thiophènecarboxamide-5 et enfin 0,8 $cm^3$ d'iodure de méthyle. On agite 1 heure 30 minutes à température ambiante (20°C environ), verse le mélange réactionnel sur 50 $cm^3$ d'eau et 60 $cm^3$ d'acétate d'éthyle. On agite 15 minutes, décante la phase organique, la lave à l'eau, la sèche sur sulfate de magnésium et l'évapore à sec sous pression réduite. Le résidu est chromatographié sur du gel de silice en utilisant un mélange cyclohexane-acétate d'éthyle (50-50 en volume) comme éluant. Les fractions contenant le produit sont rassemblées, évaporées sous pression réduite et reprises par un mélange d'eau et d'éther éthylique. La phase organique est décantée, lavée à l'eau, séchée sur sulfate de magnésium et évaporée sous pression réduite. Le résidu est agité 1 heure 30 minutes avec 20 $cm^3$ d'éther de pétrole 40-60°, filtré et séché.

On isole ainsi 1,2g de N-méthyl N-(méthyl-1 propyl) phényl-7 benzo[b]thiophènecarboxamide-5 fondant à 105°C.

Le N-(méthyl-1 propyl) phényl-7 benzo[b]thiophènecarboxamide-5 peut être préparé de la manière suivante :

On opère comme à l'exemple 1, à partir de 3g d'acide phényl-7 benzo[b]thiophènecarboxylique-5 dans 30 $cm^3$ de toluène et de 2,6 $cm^3$ de chlorure de thionyle puis de 9,94 $cm^3$ de triéthylamine et de 1,2 $cm^3$ de butanamine-2 dans 30 $cm^3$ de toluène.

Après chromatographie du résidu sur du gel de silice en utilisant comme éluant un mélange cyclohexane-acétate d'éthyle (50-50 en volume), on obtient 2,35g de N-(méthyl-1 propyl) phényl-7 benzo[b]thiophènecarboxamide-5 fondant à 195°C.

EXEMPLE 3

On opère comme à l'exemple 1, à partir de 4g d'acide phényl-4 benzo[b]thiophènecarboxylique-6 et de 3,5 $cm^3$ de chlorure de thionyle dans 40 $cm^3$ de toluène puis de 13,25 $cm^3$ de triéthylamine et de 1,6 $cm^3$ de diéthylamine dans 40 $cm^3$ de toluène.

Après chromatographie du résidu sur du gel de silice en utilisant comme éluant un mélange cyclohexane-acétate d'éthyle (50-50 en volume) puis agitation du produit recueilli dans 30 $cm^3$ d'éther de pétrole 40-60° et filtration, on isole 2,55g de N,N-diéthyl phényl-4 benzo[b]thiophènecarboxamide-6 fondant à 60°C.

L'acide phényl-4 benzo[b]thiophènecarboxylique-6 peut être préparé de la manière suivante :

On chauffe à 120°C, pendant un heure, 34,9g de phényl-5 (thiényl-2 méthylène)-3 furanone-2, 170 $cm^3$ d'acide méthanesulfonique et 170 $cm^3$ d'acide acétique glacial. On refroidit à 40°C, coule le mélange sur de la glace pilée et extrait par du chlorure de méthylène. La phase organique est lavée à l'eau et extraite par une solution normale d'hydroxyde de sodium. La phase aqueuse basique est lavée au chlorure de méthylène, acidifiée à pH 1 par addition d'une solution aqueuse d'acide chlorhydrique et extraite par du chlorure de méthylène. Le précipité qui se forme est filtré, la phase organique est séchée sur sulfate de magnésium et évaporée sous pression réduite. Le résidu d'évaporation et le précipité sont rassemblés et chromatographiés sur du gel de silice en utilisant l'acétate d'éthyle comme éluant. On isole ainsi 17,4 g d'acide phényl-4 benzo[b]thiophènecarboxylique-6 fondant à 222°C.

La phényl-5 (thiényl-2 méthylène)-3 furanone-2 peut être préparée de la façon suivante :

On chauffe à 80°C, pendant une nuit, 27,4 $cm^3$ de thiophènecarboxaldéhyde-2, 44,5 g d'acide benzoyl-3 propionique, 71 $cm^3$ d'anhydride acétique et 20,5g d'acétate de potassium fondu. On refroidit à 40°C, ajoute du chloroforme puis verse sur de la glace pilée. On extrait au chloroforme, lave la phase organique par une solution décinormale d'hydroxyde de sodium, la sèche sur sulfate de magnésium et l'évapore sous pression réduite. Le résidu est agité 45 minutes dans de l'éther de pétrole 40-60°, filtré et séché. On isole ainsi 45,3g de phényl-5 (thiényl-2 méthylène)-3 furanone-2 fondant à 141°C.

EXEMPLE 4

On opère comme à l'exemple 2, à partir de 24 $cm^3$ de diméthylsulfoxyde, de 3,6g de potasse en poudre, de 4g de N-(méthyl-1 propyl) phényl-4 benzo[b]thiophènecarboxamide-6 et de 1,6 $cm^3$ d'iodure de méthyle. Après chromatographie du résidu sur du gel de silice en utilisant un mélange cyclohexane-acétate d'éthyle (50-50 en volume) comme éluant et recristallisation dans l'éther isopropylique, on isole 1,2g de N-méthyl N-(méthyl-1 propyl) phényl-4 benzo[b]thiophènecarboxamide-6 fondant à 84°C.

Le N-(méthyl-1 propyl) phényl-4 benzo[b]thiophènecarboxamide-6 peut être préparé de la manière suivante :

On opère comme à l'exemple 1, à partir de 4 g d'acide phényl-4 benzo[b]thiophènecarboxylique-6, de 3,5 $cm^3$ de chlorure de thionyle et de 40 $cm^3$ de toluène puis de 1,59 $cm^3$ de butanamine-2 et de 13,3 $cm^3$ de triéthylamine dans 40 $cm^3$ de toluène. Ce produit présente un point de fusion de 194°C.

EXEMPLE 5

On opère comme à l'exemple 1, à partir de 2,5g d'acide phényl-7 benzo[b]thiophènecarboxylique-5 dans 25 $cm^3$ de toluène et de 2,2 $cm^3$ de chlorure de thionyle puis de 4,15 $cm^3$ de triéthylamine et de 0,97 $cm^3$ de pipéridine dans 25 $cm^3$ de toluène. Après 2 chromatographies successives du résidu sur du gel de silice, la première avec un mélange cyclohexane-éthanol-acétate d'éthyle (80-10-10) en volume) comme éluant et la deuxième avec un mélange cyclohexane-acétate d'éthyle (70-30 en volume) comme éluant, on isole un résidu que l'on agite 1 heure dans de l'éther de pétrole 40-60°. Après filtration et séchage, on isole 1,55g de [(phényl-7 benzo[b]thiényl-5) carbonyl]-1 pipéridine fondant à 112°C.

EXEMPLE 6

On opère comme à l'exemple 1, à partir de 2,5g d'acide phényl-7 benzo[b]thiophènecarboxylique-5 dans 25 $cm^3$ de toluène et de 2,2 $cm^3$ de chlorure de thionyle puis de 4,15 $cm^3$ de triéthylamine et de 1,06 $cm^3$ de N-méthylaniline dans 25 $cm^3$ de toluène.

Après chromatographie du résidu sur du gel de silice en utilisant un mélange cyclohexane-acétate d'éthyle-éthanol (80-10-10 en volume) comme éluant, on isole un résidu que l'on agite 3 heures dans de l'éther de pétrole 40-60°. Après filtration et séchage, on isole 1,9g de N-méthyl N-phényl phényl-7 benzo[b]thiophènecarboxamide-5 fondant à 127°C.

EXEMPLE 7

On opère comme à l'exemple 1, à partir de 3g d'acide phényl-7 benzo[b]thiophènecarboxylique-5 dans 30 $cm^3$ de toluène et de 2,64 $cm^3$ de chlorure de thionyle puis de 5 $cm^3$ de triéthylamine et de 1,01 $cm^3$ de N-méthyl éthylamine dans 30 $cm^3$ de toluène. Après chromatographie du résidu sur du gel de silice en utilisant un mélange cyclohexane-éthanol-acétate d'éthyle (90-5-5 en volume) comme éluant, on obtient un résidu que l'on agite dans 50 cm³ d'éther de pétrole 40-60° pendant une nuit. Après filtration et séchage, on isole 1,5g de N-éthyl N-méthyl phényl-7 benzo[b]thiophènecarboxamide-5 fondant à 93°C.

EXEMPLE 8

On opère comme à l'exemple 1, à partir de 4,67g d'acide (chloro-4 phényl)-4 benzo[b]thiophènecarboxylique-6 dans 45 cm³ de toluène et de 3,6 cm³ de chlorure de thionyle puis de 6,9 cm³ de triéthylamine et de 1,7 cm³ de diéthylamine dans 45 cm³ de toluène. Après chromatographie du résidu en utilisant un mélange cyclohexane-acétate d'éthyle (90-10 en volume) comme éluant, on obtient un résidu que l'on agite deux heures dans de l'éther de pétrole 40-60°. Après filtration et séchage, on obtient 1,07g de N,N-diéthyl (chloro-4 phényl)-4 benzo[b]thiophènecarboxamide-6 fondant à 135°C.

L'acide (chloro-4 phényl)-4 benzo[b]thiophènecarboxylique-6 peut être préparé de la manière suivante :
On opère comme à l'exemple 1 pour la préparation de l'acide phényl-7 benzo[b]thiophènecarboxylique-5, à partir d'acide méthanesulfonique (5 cm³ ), d'acide acétique (5 cm³) et de (chloro-4 phényl)-5 (thiényl-2 méthylène)-3 furanone-2 (0,0037 mole) et en chauffant à 110°C au lieu de 120°C. Il présente un point de fusion de 240°C.

La (chloro-4 phényl)-5 (thiényl-2 méthylène)-3 furanone-2 peut être préparée de la manière suivante :
On opère comme à l'exemple 1 pour la préparation de la phényl-5 (thiényl-3 méthylène)-3 furanone-2, à partir d'acide (chloro-4 benzoyl)-3 propionique (0,05 mole), de thiophènecarboxaldéhyde-2 (0,06 mole), d'anhydride acétique (14,2 cm³) et d'acétate de sodium anhydre (0,05 mole). Elle présente un point de fusion de 222°C.

EXEMPLE 9

On opère comme à l'exemple 1, à partir de 2,9g d'acide (méthoxy-4 phényl)-4 benzo[b]thiophènecarboxylique-6 dans 30 cm³ de toluène et de 2,3 cm³ de chlorure de thionyle puis de 4,3 cm³ de triéthylamine et 1,1 cm³ de diéthylamine dans 30 cm³ de toluène anhydre. Après chromatographie du résidu sur du gel de silice en utilisant un mélange cyclohexane-acétate d'éthyle (80-20 en volume) comme éluant et recristallisation dans l'éther isopropylique, on isole 1,12g de N,N-diéthyl (méthoxy-4 phényl)-4 benzo[b]thiophènecarboxamide-6 fondant à 88°C.

L'acide (méthoxy-4 phényl)-4 benzo[b]thiophènecarboxylique-6 peut être préparé de la manière suivante :
On opère comme à l'exemple 1 pour la préparation de l'acide phényl-7 benzo[b]thiophènecarboxylique-5, à partir d'acide méthanesulfonique (10 cm³ ), d'acide acétique (10 cm³) et de (méthoxy-4 phényl)-5 (thiényl-2 méthylène)-3 furanone-2 (0,007 mole) et en chauffant à 110°C au lieu de 120°C. Il présente un point de fusion de 144°C.

La (méthoxy-4 phényl)-5 (thiényl-2 méthylène)-3 furanone-2 peut être préparée de la manière suivante :
On opère comme à l'exemple 1 pour la préparation de la phényl-5 (thiényl-3 méthylène)-3 furanone-2, à partir d'acide (méthoxy-4 benzoyl)-3 propionique (0,05 mole), de thiophènecarboxaldéhyde-2 (0,06 mole), d'anhydride acétique (14,2 cm³) et d'acétate de sodium anhydre (0,05 mole). Elle présente un point de fusion de 175°C.

EXEMPLE 10

On chauffe 1 heure 30 minute, au reflux 1,3g d'acide (nitro-3 phényl)-7 benzo[b]thiophènecarboxylique-5 dans 4 cm³ de chlorure de thionyle. On évapore le chlorure de thionyle en excès sous pression réduite, ajoute au résidu 30 cm³ de toluène et 1,9 cm³ de triéthylamine, agite puis ajoute goutte à goutte 0,45 cm³ de diéthylamine. Après agitation 2 heures à température ambiante (20°C environ), le mélange réactionnel est coulé sur de l'acétate d'éthyle et une solution aqueuse de carbonate de sodium. On décante la phase organique et extrait la phase aqueuse à l'acétate d'éthyle. Les phases organiques sont rassemblées, lavées à l'eau, séchées sur sulfate de magnésium et évaporées sous pression réduite. Le résidu obtenu est chromatographié sous pression sur du gel de silice en utilisant un mélange cyclohexane-acétate d'éthyle (80-20 en volume) comme éluant. Le résidu est agité 2 heures dans de l'éther de pétrole 40-60°. Après filtration et séchage, on obtient 1,42g de N,N-diéthyl (nitro-3 phényl)-7 benzo[b]thiophènecarboxamide-5 fondant à 106°C.

L'acide (nitro-3 phényl)-7 benzo[b]thiophènecarboxylique-5 peut être préparé de la manière suivante :
On opère comme à l'exemple 1 pour la préparation de l'acide phényl-7 benzo[b]thiophènecarboxylique-5, à partir d'acide méthanesulfonique (10 cm³), d'acide acétique (10 cm³) et de (nitro-3 phényl)-5 (thiényl-3 méthylène)-3 furanone-2 (0,0062 mole) et en chauffant à 110°C au lieu de 120°C. Il se décompose vers 220°C.

La (nitro-3 phényl)-5 (thiényl-3 méthylène)-3 furanone-2 peut être préparée de la manière suivante :
On opère comme à l'exemple 1 pour la préparation de la phényl-5 (thiényl-3 méthylène)-3 furanone-2, à

partir d'acide (nitro-3 benzoyl)-3 propionique (0,023 mole), de thiophènecarboxaldéhyde-3 (0,03 mole), d'anhydride acétique (6,6 cm³ ) et d'acétate de sodium anhydre (0,023 mole) et en chauffant à 90°C au lieu de 80°C. Elle fond au dessus de 220°C en se décomposant.

EXEMPLE 11

On opère comme à l'exemple 10, à partir de 2,5g d'acide (diméthyl-3,4 phényl)-7 benzo[b]thiophènecarboxylique-5 dans 7 cm³ de chlorure de thionyle puis de 3,74 cm³ de triéthylamine et de 0,77g de N-méthyl butanamine-2 dans 30 cm³ de toluène anhydre.

Après chromatographie sous pression du résidu en utilisant un mélange cyclohexane-acétate d'éthyle (85-15 en volume) comme éluant, on isole une huile que l'on fait cristalliser dans l'éther isopropylique. On isole ainsi 1,2g de N-méthyl N-(méthyl-1 propyl) (diméthyl-3,4 phényl)-7 benzo[b]thiophènecarboxamide-5 fondant à 120°C.

L'acide (diméthyl-3,4 phényl)-7 benzo[b]thiophènecarboxylique-5 peut être préparé de la manière suivante :
On opère comme à l'exemple 1 pour la préparation de l'acide phényl-7 benzo[b]thiophènecarboxylique-5, à partir d'acide méthanesulfonique (20 cm³), d'acide acétique (20 cm³) et de (diméthyl-3,4 phényl)-5 (thiényl-3 méthylène)-3 furanone-2 (0,014 mole). Il se décompose vers 250°C.

La (diméthyl-3,4 phényl)-5 (thiényl-3 méthylène)-3 furanone-2 peut être préparée de la manière suivante :
On opère comme à l'exemple 1 pour la préparation de la phényl-5 (thiényl-3 méthylène)-3 furanone-2, à partir d'acide (diméthyl-3,4 benzoyl)-3 propionique (0,025 mole), de thiophènecarboxaldéhyde-3 (0,03 mole), d'anhydride acétique (6,6 cm³) et d'acétate de sodium anhydre (0,025 mole). Elle se décompose vers 250°C.

EXEMPLE 12

On opère comme à l'exemple 2, à partir de 2g de N-cyclopropylméthyl phényl-7 benzo[b]thiophénecarboxamide-5, de 0,8 cm³ d'iodure de méthyle et de 1,83g d'hydroxyde de potassium en poudre dans 12 cm³ diméthylsulfoxyde.

Après chromatographie sur du gel de silice en utilisant un mélange cyclohexane-acétate d'éthyle (80-20 en volume) comme éluant, on obtient 1,8g d'un résidu que l'on reprend par de l'éther éthylique. La phase organique est lavée à l'eau, séchée sur sulfate de magnésium et évaporée sous pression réduite.

On isole ainsi 1,1g de N-cyclopropylméthyl N-méthyl phényl-7 benzo[b]thiophènecarboxamide-5 dont le spectre RMN dans le chloroforme deutérié présente les caractéristiques suivantes :

$H_4$ δ : 7,6 ppm
$H_6$ δ : 7,8 ppm
autres H aromatiques δ : entre 7,3 et 8 ppm
N-$CH_3$ δ : 3,1 ppm
N-$CH_2$ δ : 3,3 ppm
-$CH_2$-<u>CH</u>- δ : 1 ppm

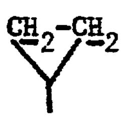 δ : entre 0 et 0,8 ppm

Le N-cyclopropylméthyl phényl-7 benzo[b]thiophènecarboxamide-5 peut être préparé de la manière suivante :
On opère comme à l'exemple 1, à partir de 5,08g d'acide phényl-7 benzo[b]thiophènecarboxylique-5, et de 4,36 cm³ de chlorure de thionyle dans 50 cm³ de toluène puis de 2,15g de chlorhydrate de cyclopropaneméthylamine et de 11,3 cm³ de triéthylamine dans 50 cm³ de toluène. Son spectre de RMN dans le chloroforme deutérié présente les caractéristiques suivantes :
$H_4$ δ : 7,8 ppm
$H_6$ δ : 8,2 ppm
autres H aromatiques δ : entre 7,4 et 7,8 ppm
NH δ : 6,5 ppm
N-$CH_2$ δ : 3,3 ppm

EXEMPLE 13

On opère comme à l'exemple 2, à partir de 1,9g de N-(méthyl-1 propyl) (chloro-2 phényl)-7 benzo[b]thiophènecarboxamide, de 0,7 cm³ d'iodure de méthyle et de 1,55g d'hydroxyde de potassium en poudre dans 12 cm³ de diméthylsulfoxyde.

Après chromatographie sur du gel de silice en utilisant un mélange cyclohexane-acétate d'éthyle (70-30 en volume) comme éluant, on isole un résidu que l'on agite dans de l'éther de pétrole 40-60°. Après filtration et séchage, on obtient 1,05g de N-méthyl N-(méthyl-1 propyl) (chloro-2 phényl)-7 benzo[b]thiophènecarboxamide-5 fondant à 98°C.

Le N-(méthyl-1 propyl) (chloro-2 phényl)-7 benzo[b]thiophènecarboxamide-5 peut être préparé de la manière suivante :
On opère comme à l'exemple 1, à partir de 2,35g d'acide (chloro-2 phényl)-7 benzo[b]thiophènecarboxylique-5 et de 1,8 $cm^3$ de chlorure de thionyle de 23 $cm^3$ de toluène puis de 0,83 $cm^3$ de butanamine-2 et de 6,9 $cm^3$ de triéthylamine dans 23 $cm^3$ de toluène. Il présente un point de fusion de 125°C.

L'acide (chloro-2 phényl)-7 benzo[b]thiophènecarboxalique-5 peut être préparé de la manière suivante :
On opère comme à l'exemple 1 pour la préparation de l'acide phényl-7 benzo[b]thiophènecarboxylique-5, à partir d'acide méthanesulfonique (22,5 $cm^3$), d'acide acétique (22,5 $cm^3$) et de (chloro-2 phényl)-5 (thiényl-3 méthylène)-3 furanone-2 (0,016 mole). Il présente un point de fusion de 225°C.

La (chloro-2 phényl)-5 (thiényl-3 méthylène)-3 furanone-2 peut être préparée de la manière suivante :
On opère comme à l'exemple 1 pour la préparation de la phényl-5 (thiényl-3 méthylène)-3 furanone-2, à partir d'acide (chloro-2 benzoyl)-3 propionique (0,017 mole), de thiophènecarboxaldéhyde-3 (0,021 mole), d'anhydride acétique (4,9 $cm^3$) et d'acétate de sodium anhydre (0,17 mole).

EXEMPLE 14

On opère comme à l'exemple 2, à partir de 2,65g de N-(méthyl-1 propyl) (fluoro-2 phényl)-7 benzo[b]thiophènecarboxamide-5, de 1 $cm^3$ d'iodure de méthyle et de 2,3g d'hydroxyde de potassium en poudre dans 16 $cm^3$ de diméthylsulfoxyde.

Après chromatographie sur du gel de silice en utilisant un mélange cyclohexane-acétate d'éthyle (70-30 en volume) comme éluant, on isole un résidu que l'on agite 30 minutes dans de l'éther de pétrole 40-60°. Après filtration et séchage, on obtient 0,85g de N-méthyl N-(méthyl-1 propyl) (fluoro-2 phényl)-7 benzo[b]thiophènecarboxamide-5 fondant à 89°C.

Le N-(méthyl-1 propyl) (fluoro-2 phényl)-7 benzo[b]thiophènecarboxamide-5 peut être préparé de la manière suivante :
On opère comme à l'exemple 1, à partir de 3,2g d'acide (fluoro-2 phényl)-7 benzo[b]thiophènecarboxylique-5 et de 2,6 $cm^3$ de chlorure de thionyle dans 32 $cm^3$ de toluène puis de 1,2 $cm^3$ de butanamine-2 et de 9,9 $cm^3$ de triéthylamine dans 32 $cm^3$ de toluène. Il présente un point de fusion de 185°C.

L'acide (fluoro-2 phényl)-7 benzo[b]thiophènecarboxylique-5 peut être préparé de la manière suivante :
On opère comme à l'exemple 1 pour la préparation de l'acide phényl-7 benzo[b]thiophènecarboxylique-5, à partir d'acide méthanesulfonique (44 $cm^3$), d'acide acétique (44 $cm^3$) et de (fluoro-2 phényl)-5 (thiényl-3 méthylène)-3 furanone-2 (0,032 mole). Il présente un point de fusion de 223°C.

La (fluoro-2 phényl)-5 (thiényl-3 méthylène)-3 furanone-2 peut être préparée de la manière suivante :
On opère comme à l'exemple 1 pour la préparation de la phényl-5 (thiényl-3 méthylène)-3 furanone-2, à partir d'acide (fluoro-2 benzoyl)-3 propionique (0,037 mole), de thiophènecarboxaldéhyde-3 (0,045 mole), d'anhydride acétique (10,5 $cm^3$) et d'acétate de sodium anhydre (0,037 mole). Il présente un point de fusion de 88°C.

EXEMPLE 15

On opère comme à l'exemple 1, à partir de 2g d'acide (trifluorométhyl-3 phényl)-7 benzo[b]thiophènecarboxylique-5 dans 20 $cm^3$ de toluène et de 1,4 $cm^3$ de chlorure de thionyle puis de 0,77g de chlorhydrate de N-méthylbutanamine-2 et de 6,1 $cm^3$ de triéthylamine dans 20 $cm^3$ de toluène.

Après deux chromatographies successives sur du gel de silice, la première au moyen d'un mélange cyclohexane-acétate d'éthyle(70-30 en volume) comme éluant, et la deuxième au moyen d'un mélange cyclohexane-éther éthylique (70-30 en volume) comme éluant on obtient 1,1g de N-méthyl N-(méthyl-1 propyl) (trifluorométhyl-3 phényl)-7 benzo[b]thiophènecarboxamide-5 sous forme d'une huile jaune et dont le spectre RMN dans le chloroforme deutérié présente les caratéristiques suivantes :

$H_4$ δ : 7,6 ppm
$H_6$ δ : 7,9 ppm
autres H aromatiques δ : entre 7,3 et 8 ppm

$N-CH_3$ δ : 2,9 ppm
$N-CH-\underline{CH_3}$ δ : 1,2 ppm
$N-CH-\underline{CH_2}-CH_3$ δ : 1,6 ppm
$N-CH-CH_2-\underline{CH_3}$ δ : 0,8 ppm

L'acide (trifluorométhyl-3 phényl)-7 benzo[b]thiophènecarboxylique-5 peut être préparé de la manière suivante :
On opère comme à l'exemple 1 pour la préparation de l'acide phényl-7 benzo[b]thiophènecarboxylique-5, à partir d'acide méthanesulfonique (15 $cm^3$), d'acide acétique (15 $cm^3$) et de (trifluorométhyl-3 phényl)-5 (thiényl-3 méthylène)-3 furanone-2 (0,0092 mole). Il présente un point de fusion de 150°C.

La (trifluorométhyl-3 phényl)-5 (thiényl-3 méthylène)-3 furanone-2 peut être préparée de la manière suivante :
On opère comme à l'exemple 1 pour la préparation de la phényl-5 (thiényl-3 méthylène)-3 furanone-2, à partir d'acide (trifluorométhyl-3-benzoyl)-3 propionique (0,012 mole), de thiophènecarboxaldéhyde-3 (0,0146 mole), d'anhydride acétique (3,45 $cm^3$) et d'acétate de sodium anhydre (0,012 mole). Elle présente un point de fusion de 192°C.

## EXEMPLE 16

On opère comme à l'exemple 1, à partir de 1g d'acide phényl-4 benzo[b]furannecarboxylique-6 dans 10 $cm^3$ de toluène et de 0,92 $cm^3$ de chlorure de thionyle puis de 3,5 $cm^3$ de triéthylamine et de 0,43 $cm^3$ de diéthylamine dans 10 $cm^3$ de toluène.

Après deux chromatographies successives sur du gel de silice, la première au moyen d'un mélange cyclohexane-éthanol-acétate d'éthyle (80-10-10 en volume) comme éluant et la deuxième au moyen d'un mélange cyclohexane-éthanol-acétate d'éthyle (92-4-4 en volume) comme éluant, on obtient un résidu (0,6g) que l'on chromatographie sous pression sur du gel de silice en utilisant un mélange cyclohexane-acétate d'éthyle (85-15 en volume) comme éluant. On obtient 0,32g de N,N-diéthyl phényl-4 benzo[b]furannecarboxamide-6 sous forme d'une huile épaisse et dont le spectre de RMN du proton dans le chloroforme deuterié présente les caractéristiques suivantes :

$H_2$ δ : 7,8 ppm
$H_3$ δ : 6,9 ppm
autres H aromatiques δ : entre 7,4 et 7,7 ppm
$CH_3$ δ : 1,2 ppm
$CH_2$ δ : 3,4 ppm

L'acide phényl-4 benzo[b]furannecarboxylique-6 peut être préparé de la manière suivante :
On opère comme à l'exemple 1 pour la préparation de l'acide phényl-7 benzo[b]thiophènecarboxylique-5, à partir d'acide méthane sulfonique(600 $cm^3$), d'acide acétique (600 $cm^3$) et de (furyl-2 méthylène)-3 phényl-5 furanone-2 (0,521 mole). Il présente un point de fusion de 180°C.

La (furyl-2 méthylène)-3 phényl-5 furanone-2 peut être préparée de la manière suivante :
On opère comme à l'exemple 1 pour la préparation de la phényl-5 (thiényl-3 méthylène)-3 furanone-2, à partir d'acide benzoyl-3 propionique(0,5 mole), de furannecarboxaldéhyde-2 (0,6 mole), d'anhydride acétique (142 $cm^3$) et d'acétate de sodium anhydre (0,5 mole). Elle présente un point de fusion de 117°C.

## EXEMPLE 17

On opère comme à l'exemple 1, à partir de 1,1g d'acide phényl-4 benzo[b]furannecarboxylique-6 dans 11 $cm^3$ de toluène et de 1,01 $cm^3$ de chlorure de thionyle puis de 4,6 $cm^3$de triéthylamine et de 0,57g de N-méthylbutanamine-2 dans 11 $cm^3$ de toluène.

Après deux chromatographies successives sur du gel de silice, la première au moyen d'un mélange cyclohexane-acétate d'éthyle-éthanol (80-10-10 en volume) comme éluant et la deuxième au moyen d'un mélange cyclohexane-éthanol-acétate d'éthyle (92-4-4 en volume) comme éluant, on obtient un résidu (0,6g) que l'on chromatographie sous pression sur du gel de silice en utilisant un mélange cyclohexane-acétate d'éthyle (85-15 en volume) comme éluant.
On obtient 0,35g de N-méthyl N-(méthyl-1 propyl) phényl-4 benzo[b]furannecarboxamide-6 sous forme d'huile et dont le spectre de RMN du proton dans le chloroforme deutérié présente les caractéristiques suivantes :

$H_2$ δ : 7,8 ppm
$H_3$ δ : 6,9 ppm

autres H aromatiques δ : entre 7,4 et 7,7 ppm
N-$CH_3$ δ : 2,9 ppm
CH-<u>$CH_3$</u> δ : 1,2 ppm
<u>CH</u>-$CH_3$ δ : 4,8 et 3,8 ppm
<u>$CH_2$</u>-$CH_3$ δ : 1,5 ppm
$CH_2$-<u>$CH_3$</u> δ : 0,8 ppm

EXEMPLE 18

On opère comme à l'exemple 1, à partir de 4g d'acide phényl-7 benzo[b]furannecarboxylique-5 dans 20 $cm^3$ de toluène et de 7,4 $cm^3$ de chlorure de thionyle puis de 1,7 $cm^3$ de diéthylamine et de 14,1 $cm^3$ de triéthylamine dans 40 $cm^3$ de toluène.

Après deux chromatographies successives sur du gel de silice, la première au moyen d'un mélange cyclohexane-éthanol-acétate d'éthyle (90-5-5 en volume) comme éluant, la deuxiène au moyen d'un mélange cyclohexane-acétate d'éthyle (80-20 en volume) comme éluant, on isole un résidu que l'on agite une heure dans de l'éther de pétrole 40-60°.

Après filtration et séchage, on obtient 2,75g de N,N-diéthyl phényl-7 benzo[b]furannecarboxamide-5 fondant à 56°C.

L'acide phényl-7 benzo[b]furannecarboxylique-5 peut être préparé de la manière suivante :
On opère comme à l'exemple 1 pour la préparation de l'acide phényl-7 benzo[b]thiophènecarboxylique-5, à partir d'acide méthanesulfonique (31 $cm^3$), d'acide acétique (31 $cm^3$) et de (furyl-3 méthylène)-3 phényl-5 furanone-2 (0,0263 mole). Il présente un point de fusion de 197°C.

La (furyl-3 méthylène)-3 phényl-5 furanone-2 peut être préparée de la manière suivante :
On opère comme à l'exemple 1 pour la préparation de la phényl-5 (thiényl-3 méthylène)-3 furanone-2, à partir d'acide benzoyl-3 propionique (0,23 mole), de furannecarboxaldéhyde-3 (0,276 mole), d'anhydride acétique (65,2 $cm^3$) et d'acétate de sodium anhydre (0,23 mole). Elle présente un point de fusion de 174°C.

EXEMPLE 19

On opère comme à l'exemple 2, à partir de 3g de N-(méthyl-1 propyl) phényl-7 benzo[b]furannecarboxamide-5, de 1,9 $cm^3$ d'iodure de méthyle et de 2,9g d'hydroxyde de potassium en poudre dans 18 $cm^3$ de diméthylsulfoxyde.

Après deux chromatographies successives sur du gel de silice, la première au moyen d'un mélange cyclohexane-acétate d'éthyle (70-30 en volume) comme éluant et la deuxième au moyen d'éther éthylique comme éluant, on obtient 2,55g de N-méthyl N-(méthyl-1 propyl) phényl-7 benzo[b]furannecarboxamide-5 dont le spectre de RMN du proton dans le chloroforme deutérié présente les caractéristiques suivantes:

$H_2$ δ : 7,8 ppm
$H_3$ δ : 6,9 ppm
autres H aromatiquesδ : entre 7,3 et 8 ppm
N-$CH_3$ δ : 2,9 ppm CH-<u>$CH_3$</u> δ : 1,2 ppm
<u>CH</u>-$CH_3$ δ : 4,8 et 3,8 ppm
<u>$CH_2$</u>-$CH_3$ δ : 1,5 ppm
$CH_2$-<u>$CH_3$</u> δ : 0,9 ppm

Le N-(méthyl-1 propyl) phényl-7 benzo[b]furannecarboxamide-5 peut être préparé de la manière suivante :
On opère comme à l'exemple 1, à partir de 8g d'acide phényl-7 benzo[b]furannecarboxylique-5 et de 14,8 $cm^3$ de chlorure de thionyle dans 40 $cm^3$ de toluène puis de 3,4 $cm^3$ de butanamine-2 et de 17,2 $cm^3$ de triéthylamine dans 80 $cm^3$ de toluène.

EXEMPLE 20

On opère comme à l'exemple 1, à partir de 3,5g d'acide (thiényl-2)-7 benzo[b]furannecarboxylique-5 dans 17,5 $cm^3$ de toluène et de 3,15 $cm^3$ de chlorure de thionyle puis de 12 $cm^3$ de triéthylamine et de 1,47 $cm^3$ de diéthylamine dans 35 $cm^3$ de toluène.

Après chromatographie sur du gel de silice en utilisant un mélange cyclohexane-acétate d'éthyle (70-30 en volume) comme éluant, on obtient une huile qui cristallise dans l'éther de pétrole. On isole ainsi 1,8g de N,N-diéthyl (thiényl-2)-7 benzo[b]furannecarboxamide-5 fondant à 64°C.

L'acide (thiényl-2)-7 benzo[b]furannecarboxylique-5 peut être préparé de la manière suivante :
On opère comme à l'exemple 1 pour la préparation de l'acide phényl-7 benzo[b]thiophènecarboxylique-5, à partir d'acide méthanesulfonique (90 $cm^3$), d'acide acétique (90 $cm^3$) et de (furyl-3 méthylène)-3 (thiényl-2)-5 furanone-2 (0,071 mole). Il présente un point de fusion de 192°C.

La (furyl-3 méthylène)-3 (thiényl-2)-5 furanone-2 peut être préparée de la manière suivante :
On opère comme à l'exemple 1 pour la préparation de la phényl-5 (thiényl-3 méthylène)-3 furanone-2, à partir de l'acide oxo-4 (thiényl-2)-4 butyrique (0,102 mole), de furannecarboxaldéhyde-3 (0,122 mole), d'anhydride acétique (29 $cm^3$) et d'acétate de sodium anhydre (0,102 mole). Elle présente un point de fusion de 114°C.

EXEMPLE 21

On opère comme à l'exemple 1, à partir de 4g d'acide (thiényl-2)-7 benzo[b]furannecarboxylique-5 dans 20 $cm^3$ de toluène et de 3,6 $cm^3$ de chlorure de thionyle puis de 16,1 $cm^3$ de triéthylamine et de 2,2g de chlorhydrate de N-methylbutanamine-2 dans 40 $cm^3$ de toluène.

Après chromatographie sur du gel de silice en utilisant un mélange cyclohexane-acétate d'éthyle (70-30 en volume) comme éluant, on isole 1,5g de N-méthyl N-(méthyl-1 propyl) (thiényl-2)-7 benzo[b]furannecarboxamide-5 fondant à 96°C.

Les médicaments selon l'invention sont constitués par un composé de formule (I) où lorsque le groupe $NR_1R_2$ comporte au moins un atome de carbone asymétrique, ses racémiques ou ses stéréoisomères, à l'état pur ou sous forme d'une composition dans laquelle il est associé à tout autre produit pharmaceutiquement compatible, pouvant être inerte ou physiologiquement actif. Ces médicaments selon l'invention peuvent être utilisés par voie orale, parentérale, rectale ou topique.
Comme compositions solides pour administration orale peuvent être utilisés des comprimés, pilules, poudres (capsules de gélatine, cachets) ou granulés. Dans ces compositions, le principe actif selon l'invention est mélangé à un ou plusieurs diluants inertes, tels que amidon, cellulose, saccharose, lacrose ou silice. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississsants, aromatisants ou stabilisants.

Les compositions stériles pour administration parentérale peuvent être de préférence des solutions non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables.

Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le produit actif des excipients tels que le beurre de cacao, des glycérides semi-synthétiques ou des polyéthyleneglycols.

Les compositions pour administraton topique peuvent être par exemple des crèmes, pommades, lotions, collyres, collutoires, gouttes nasales ou aérosols.

En thérapeutiques humaine, les composés selon l'invention sont particulièrement utiles comme anxiolytiques, antiangoreux et immunomodulateurs.

Les doses dépendent de l'effet recherché, de la durée du traitement et de la voie d'administration utilisée ; elles sont généralement comprises entre 10 et 500 mg par jour par voie orale pour un adulte avec des doses unitaires allant de 2 à 100 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

Exemple A

On prépare, selon la technique habituelle, des gélules dosées à 50 mg de produit actif ayant la composition suivante :

| | |
|---|---|
| – N-méthyl N-(méthyl-1 propyl) phényl-4 benzo[b]thiophènecarboxamide-6 | 50 mg |
| – Cellulose | 18 mg |
| – Lactose | 55 mg |
| – Silice colloïdale | 1 mg |
| – Carboxyméthylamidon sodique | 10 mg |
| – Talc | 10 mg |
| – Stéarate de magnésium | 1 mg |

Exemple B

On prépare selon la technique habituelle, des comprimés dosés à 50 mg de produit actif ayant la composition suivante :

| | |
|---|---|
| – N-méthyl N-(méthyl-1 propyl) (chloro-2 phényl)-7 benzo[b]thiophènecarboxamide-5 | 50 mg |
| – Lactose | 104 mg |
| – Cellulose | 40 mg |
| – Polyvidone | 10 mg |
| – Carboxyméthylamidon sodique | 22 mg |
| – Talc | 10 mg |
| – Stéarate de magnésium | 2 mg |
| – Silice colloïdale | 2 mg |
| – Mélange d'hydroxyméthylcellulose, glycérine, oxyde de titane (72–3,5–24,5) | q.s.p. 1 comprimé pelliculé terminé à 245 mg |

Exemple C

On prépare une solution injectable contenant 10 mg de produit actif ayant la composition suivante :

| | |
|---|---|
| – N-méthyl N-(méthyl-1 propyl) phényl-4 benzo[b]furannecarboxamide-6 | 10 mg |
| – Acide benzoïque | 80 mg |
| – Alcool benzylique | 0,06 $cm^3$ |
| – Benzoate de sodium | 80 mg |
| – Ethanol à 95% | 0,4 $cm^3$ |
| – Hydroxyde de sodium | 24 mg |
| – Propylène glycol | 1,6 $cm^3$ |
| – Eau | q.s.p 4 $cm^3$ |

**Revendications pour les états contractants: BE, CH, DE, SE, FR, GB, IT, LI, LU, NL**

1 - Composés de formule :

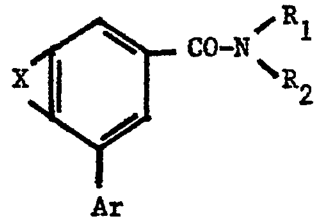

(I)

dans laquelle $R_1$ et $R_2$, identiques ou différents, représentent des groupes alkyle à chaîne droite ou ramifiée comportant 1 à 4 atomes de carbone, cycloalkylalkyle dont la partie alkyle comporte 1 à 3 atomes de carbone et la partie cycloalkyle comporte 3 à 6 atomes de carbone ou phényle, $R_1$ et $R_2$ peuvent également former ensemble avec l'atome d'azote auquel ils sont rattachés un cycle pipéridine,
Ar représente un radical phényle, thiényle ou phényle substitué par un ou deux substituants choisis parmi les atomes d'halogène, les groupes alkyle ou alcoxy comportant 1 à 4 atomes de carbone, nitro ou trifluorométhyle et,

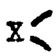 représente l'un des enchaînements suivants:

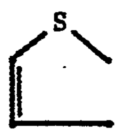 , 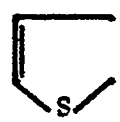 , 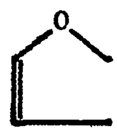 , 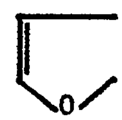

et lorsque le groupe $NR_1R_2$ comporte au moins un atome de carbone asymétrique, leurs racémiques et stéréoisomères.

2 - Composés de formule (I) selon la revendication 1 pour lesquels $R_1$ et $R_2$, identiques ou différents sont des groupes alkyle à chaîne droite ou ramifiée comportant 1 à 4 atomes de carbone et Ar est un radical phényle éventuellement substitué par un atome d'halogène ou un groupe nitro ou un radical thiényl-2 et lorsque le groupe $NR_1R_2$ comporte au moins un atome de carbone asymétrique, leurs racémiques et stéréoisomères.

3 - Procédé de préparation d'un composé selon la revendication 1 caractérisé en ce que l'on fait réagir une amine de formule :

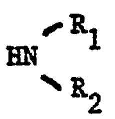

(VI)

sur un composé de formule :

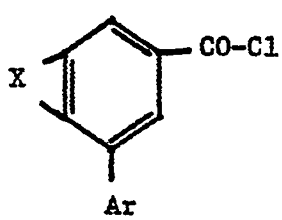

(VII)

dans lesquelles $R_1$, $R_2$ Ar, 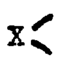
ont les mêmes significations que dans la formule (I) et isole le produit.

4 - Procédé de préparation d'un composé selon la revendication 1 pour lequel $R_1$ représente un groupe alkyle comportant 1 à 4 atomes de carbone caractérisé en ce que l'on alkyle un dérivé de formule :

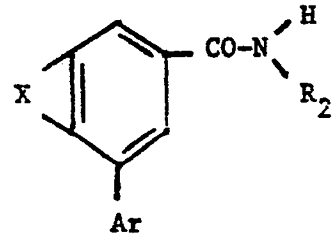

(XI)

dans laquelle $R_2$, Ar et 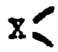 ont les mêmes significations que dans la formule (I), avec un halogènure d'alkyle de formule Hal-$R'_1$ (XII) dans laquelle Hal est un atome d'halogène et $R'_1$ est un groupe alkyle comportant 1 à 4 atomes de carbone et isole le produit.

5 - Médicaments caractérisés en ce qu'ils contiennent au moins un composé selon la revendication 1, éventuellement en association avec un ou plusieurs diluants ou adjuvants compatibles et pharmaceutiquement acceptables.

6 - Médicaments caractérisés en ce qu'ils contiennent au moins un composé selon la revendication 2, éventuellement en association avec un ou plusieurs diluants ou adjuvants compatibles et pharmaceutiquement acceptables.

**Revendication pour les états contractants: AT, ES, GR**

1 - Procédé de préparation des composés de formule :

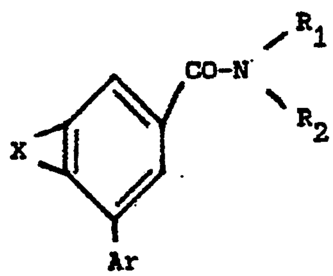

(I)

dans laquelle $R_1$ et $R_2$, identiques ou différents, représentent des groupes alkyle à chaîne droite ou ramifiée comportant 1 à 4 atomes de carbone, cycloalkylalkyle dont la partie alkyle comporte 1 à 3 atomes de carbone et la partie cycloalkyle comporte 3 à 6 atomes de carbone ou phényle, $R_1$ et $R_2$ peuvent également former ensemble avec l'atome d'azote auquel ils sont rattachés, un cycle pipéridine,
Ar représente un radical phényle, thiényle ou phényle substitué par un ou deux substituants choisis parmi les atomes d'halogène, les groupes alkyle ou alcoxy comportant 1 à 4 atomes de carbone, nitro ou trifluorométhyle et

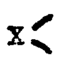 représente l'un des enchaînements suivants :

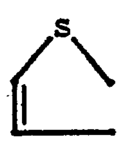 , 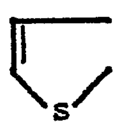 , 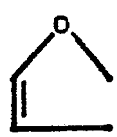 , 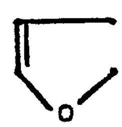

et lorsque le groupe $NR_1R_2$ comporte au moins un atome de carbone asymétrique, leurs racémiques et stéréoisomères caractérisé en ce que :

A - pour les composés de formule (I) dans laquelle $R_1$, $R_2$, Ar et 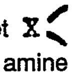 ont les significations mentionnées ci-dessus, on fait réagir une amine de formule :

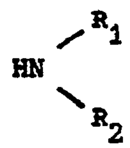

(VI)

sur un composé de formule :

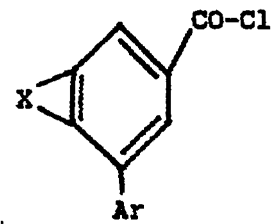

(VII)

dans lesquelles $R_1$, $R_2$, Ar et 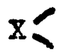 ont les mêmes significations que dans la formule (I) et isole le produit ;
B - pour les composés pour lesquels $R_1$ représente un groupe alkyle comportant 1 à 4 atomes de carbone et $R_2$, Ar et 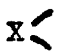 ont les mêmes significatons que ci-dessus, on alkyle un dérivé de formule :

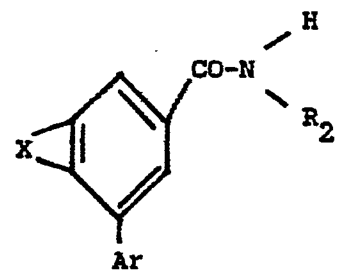

(XI)

dans laquelle $R_2$, Ar et 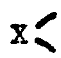 ont les mêmes significations que dans la formule (I), avec un halogènure d'alkyle de formule :
Hal-$R'_1$ (XII)
dans laquelle Hal est un atome d'halogène et $R'_1$ est un groupe alkyle comportant 1 à 4 atomes de carbone et isole le produit.

**Patentansprüche für die Vertragsstaaten: BE, CH, DE, FR, GB, IT, LI, LU, NL, SE**

1. Verbindungen der Formel:

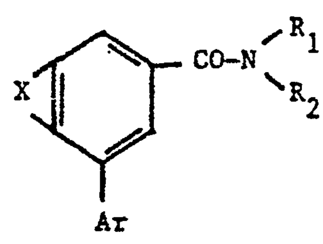

(I)

worin $R_1$ und $R_2$, die identisch oder verschieden sind, bedeuten Alkylgruppen mit gerader oder verzweigter Kette mit 1 bis 4 Kohlenstoffatomen, Cycloalkylalkyl, dessen Alkylteil 1 bis 3 Kohlenstoffatome hat und der Cycloalkylteil 3 bis 6 Kohlenstoffatome hat, oder Phenyl, wobei $R_1$ und $R_2$ auch zusammen mit dem Stickstoffatom, an das sie gebunden sind, einen Piperidinring bilden können,
Ar einen Phenyl-, Thienyl- oder Phenylrest, substituiert durch einen oder zwei Substituenten, bedeutet, ausgewählt unter den Halogenatomen, den Alkyl- oder Alkoxygruppen mit 1 bis 4 Kohlenstoffatomen, Nitro oder Trifluormethyl und

X< eine der folgenden Verkettungen bedeutet:

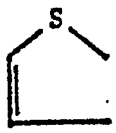 , 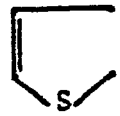 , 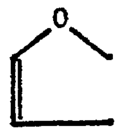 , 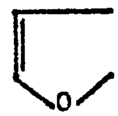

und, falls die Gruppe $NR_1R_2$ mindestens ein asymmetrisches Kohlenstoffatom trägt, ihre Racemate und Stereoisomeren.

2. Verbindungen der Formel (I) gemäß Anspruch 1, bei denen $R_1$ und $R_2$, die identisch oder verschieden sind, Alkylgruppen mit gerader oder verzweigter Kette mit 1 bis 4 Kohlenstoffatomen sind und Ar ein Phenylrest ist, der gegebenenfalls durch ein Halogenatom oder eine Nitrogruppe substituiert ist, oder ein Thienyl-2-Rest ist und, wenn die Gruppe $NR_1R_2$ mindestens ein asymmetrisches Kohlenstoffatom trägt, ihre Racemate und Stereoisomeren.

3. Verfahren zur Herstellung einer Verbindung gemäß Anspruch 1, dadurch gekennzeichnet, daß man ein Amin der Formel:

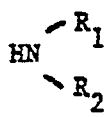 (VI)

mit einer Verbindung der Formel:

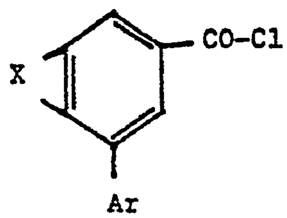 (VII)

worin $R_1$, $R_2$, Ar, X<

dieselben Bedeutungen wie in Formel (I) haben, zur Reaktion bringt und das Produkt isoliert.

4. Verfahren zur Herstellung einer Verbindung gemäß Anspruch 1, worin $R_1$ eine Alkylgruppe mit 1 bis 4 Kohlenstoffatomen bedeutet, dadurch gekennzeichnet, daß man ein Derivat der Formel:

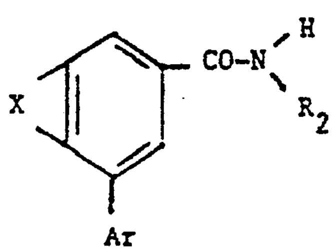 (XI)

worin $R_2$, Ar und X<

dieselben Bedeutungen wie in Formel (I) haben, mit einem Alkylhalogenid der Formel:

Hal–R'$_1$ (XII)

alkyliert, worin Hal ein Halogenatom ist und R'$_1$ eine Alkylgruppe mit 1 bis 4 Kohlenstoffatomen darstellt, und das Produkt isoliert.

5. Arzneimittel, dadurch gekennzeichnet, daß sie mindestens eine Verbindung gemäß Anspruch 1, gegebenenfalls zusammen mit einem oder mehreren verträglichen und pharmazeutisch annehmbaren Verdünnungs- oder Hilfsmitteln, enthalten.

6. Arzneimittel, dadurch gekennzeichnet, daß sie wenigstens eine Verbindung gemäß Anspruch 2, gegebenenfalls zusammen mit einem oder mehreren verträglichen und pharmazeutisch annehmbaren Verdünnungs- oder Hilfsmitteln, enthalten.

**Patentansprüche für die Vertragsstaaten: AT, ES, GR**

1. Verfahren zur Herstellung von Verbindungen der Formel:

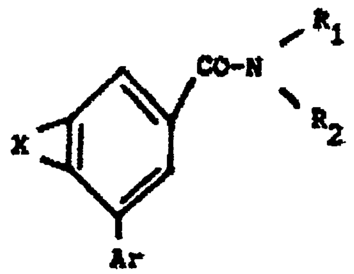

(I)

in welcher $R_1$ und $R_2$ unabhängig voneinander Alkylgruppen mit gerader oder verzweigter Kette und 1 bis 4 Kohlenstoffatomen, Cycloalkylalkylgruppen mit 1 bis 3 Kohlenstoffatomen im Alkylteil und 3 bis 6 Kohlenstoffatomen im Cycloalkylteil oder Phenylgruppen darstellen, wobei $R_1$ und $R_2$ auch zusammen mit dem Stickstoffatom, an das sie gebunden sind, einen Piperidinring bilden können,
Ar einen Phenyl-, Thienyl- oder einen durch einen oder zwei Substituenten, ausgewählt aus den Halogenatomen, den Alkyl- oder Alkoxygruppen mit 1 bis 4 Kohlenstoffatomen, Nitro- oder Trifluormethylgruppen, substituierten Phenylrest darstellt und
X< eine der folgenden Gruppierungen darstellt:

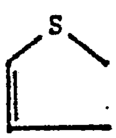 , 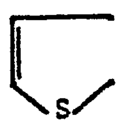 , 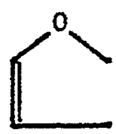 , 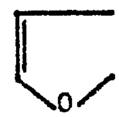

und, wenn die Gruppe $NR_1R_2$ zumindest ein asymmetrisches Kohlenstoffatom enthält, von ihren racemischen und stereoisomeren Formen, dadurch gekennzeichnet, daß man:
A – für die Verbindungen der Formel (I), worin $R_1$, $R_2$, Ar und
X< die obigen Bedeutungen haben, ein Amin der Formel:

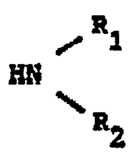

(VI)

mit einer Verbindung der Formel:

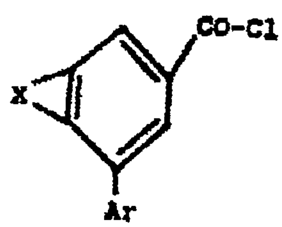

(VII)

in welchen $R_1$, $R_2$, Ar und X<
die gleichen Bedeutungen wie in Formel (I) haben, reagieren läßt und das Produkt isoliert;
B – für die Verbindungen, worin $R_1$ eine Alkylgruppe mit 1 bis 4 Kohlenstoffatomen darstellt und
$R_2$, Ar und X< die obigen Bedeutungen haben, eine Verbindung der Formel:

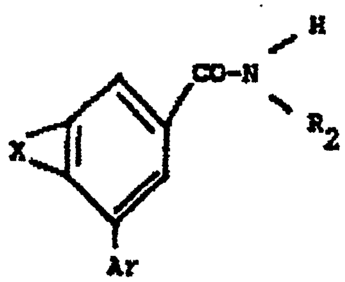

(XI)

in welcher $R_2$, Ar und 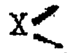

die gleichen Bedeutungen wie in der Formel (I) haben, mit einem Alkylhalogenid der Formel:

Hal–$R'_1$ (XII)

in welcher Hal ein Halogenatom ist und $R'_1$ eine Alkylgruppe mit 1 bis 4 Kohlenstoffatomen ist, alkyliert und das Produkt isoliert.

**Claims for the Contracting States: BE, CH, DE, FR, GB, IT, LI, LU, NL, SE**

1. Compounds of formula:

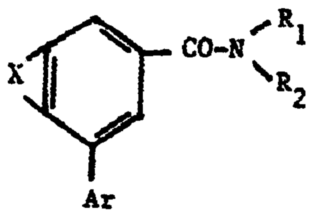

(I)

in which $R_1$ and $R_2$, which may be identical or different, represent straight- or branched-chain alkyl groups containing 1 to 4 carbon atoms, cycloalkylalkyl groups in which the alkyl part contains 1 to 3 carbon atoms and the cycloalkyl part contains 3 to 6 carbon atoms or phenyl groups; $R_1$ and $R_2$, together with the nitrogen atom to which they are attached, may also form a piperidine ring,
Ar represents a phenyl or thienyl radical, or a phenyl radical substituted by one or two substituents chosen from halogen atoms, alkyl or alkoxy groups containing 1 to 4 carbon atoms, or nitro or trifluoromethyl groups, and

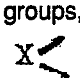 represents one of the following linkages:

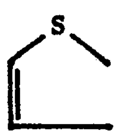 , 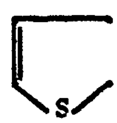 , 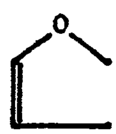 , 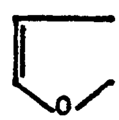

and when the group $NR_1R_2$ contains at least one asymmetric carbon atom, their racemic forms and stereoisomers.

2. Compounds of formula (I) according to claim 1, in which $R_1$ and $R_2$, which may be identical or different, are straight- or branched-chain alkyl groups containing 1 to 4 carbon atoms and Ar is a phenyl radical optionally substituted by a halogen atom or a nitro group or a 2-thienyl radical and, when the group $NR_1R_2$ contains at least one asymmetric carbon atom, their racemic forms and stereoisomers.

3. Process for the preparation of a compound according to claim 1, characterized in that an amine of formula:

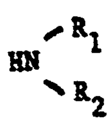

(VI)

is reacted with a compound of formula:

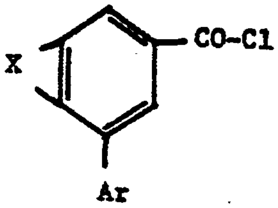

(VII)

in which $R_1$, $R_2$, Ar and 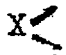 have the same meanings as in formula (I) and the product is isolated.

4. Process for the preparation of a compound according to claim 1, in which $R_1$ represents an alkyl group containing 1 to 4 carbon atoms, characterized in that a derivative of formula:

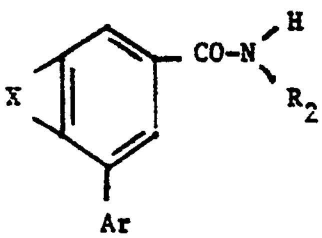

(XI)

in which $R_2$, Ar and 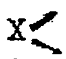 have the same meanings as in formula (I) is alkylated with an alkyl halide of formula:

Hal–R'$_1$ (XII)

in which Hal is a halogen atom and R'$_1$ is an alkyl group containing 1 to 4 carbon atoms and the product is isolated.

5. Medicinal products characterized in that they contain at least one compound according to claim 1, optionally in combination with one or more compatible and pharmaceutically acceptable diluents or adjuvants.

6. Medicinal products characterized in that they contain at least one compound according to claim 2, optionally in combination with one or more compatible and pharmaceutically acceptable diluents or adjuvants.

**Claims for the Contracting States: AT, ES, GR**

1. Process for the preparation of compounds of formula:

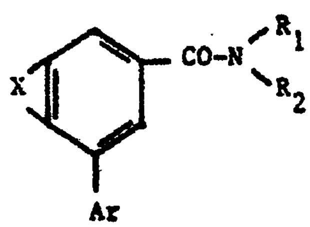

(I)

in which $R_1$ and $R_2$, which may be identical or different, represent straight- or branched-chain alkyl groups containing 1 to 4 carbon atoms, cycloalkylalkyl groups in which the alkyl part contains 1 to 3 carbon atoms and the cycloalkyl part contains 3 to 6 carbon atoms or phenyl groups; $R_1$ and $R_2$, together with the nitrogen atom to which they are attached, may also form a piperidine ring,
Ar represents a phenyl or thienyl radical, or a phenyl radical substituted with one or two substituents chosen from amongst halogen atoms, alkyl or alkoxy groups containing 1 to 4 carbon atoms, or nitro or trifluoromethyl groups, and

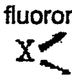 represents one of the following linkages:

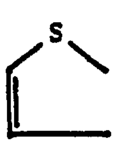 , 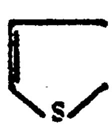 , 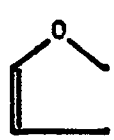 , 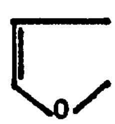

and when the group $NR_1R_2$ contains at least one asymmetric carbon atom, their racemic forms and stereoisomers, characterized in that:

A – in the case of the compounds of formula (I) in which $R_1$, $R_2$ Ar and X< have the meanings mentioned above, an amine of formula:

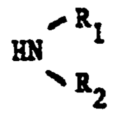

(VI)

is reacted with a compound of formula:

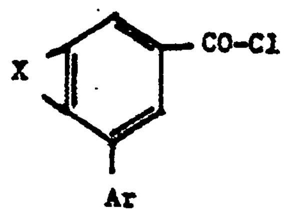

(VII)

in which $R_1$, $R_2$, Ar and X< have the same meanings as in formula (I) and the product is isolated;

B – in the case of the compounds in which $R_1$ denotes an alkyl group containing 1 to 4 carbon atoms and $R_2$, Ar and X< have the same meanings as above, a derivative of formula:

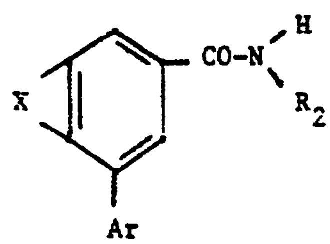

(XI)

in which $R_2$, Ar and X< have the same meanings as in formula (I) is alkylated with an alkyl halide of formula:

Hal–$R'_1$ (XII)

in which Hal is a halogen atom and $R'_1$ is an alkyl group containing 1 to 4 carbon atoms and the product is isolated.